(19) Europäisches Patentamt
European Patent Office
Office européen des brevets

(11) Veröffentlichungsnummer: **0 089 653**
**A2**

# (12) EUROPÄISCHE PATENTANMELDUNG

(21) Anmeldenummer: 83102734.7

(22) Anmeldetag: 19.03.83

(51) Int. Cl.$^3$: C 07 C 139/14
C 07 C 139/04, C 07 C 143/02

(30) Priorität: 23.03.82 DE 3210573

(43) Veröffentlichungstag der Anmeldung: 28.09.83 Patentblatt 83/39

(84) Benannte Vertragsstaaten: AT BE CH DE FR GB IT LI NL SE

(71) Anmelder: HOECHST AKTIENGESELLSCHAFT
Postfach 80 03 20
D-6230 Frankfurt am Main 80(DE)

(72) Erfinder: Mees, Bernhard, Dr.
Crooked Oak Lane 4700
Charlotte North Carolina 28211(US)

(72) Erfinder: Ramloch, Herbert, Dr.
Eichhornweg 3
D-6232 Bad Soden am Taunus(DE)

## (54) Verfahren zum Abtrennen von Schwefelsäure aus dem bei der Sulfoxidation von Paraffinen anfallenden Reaktionsgemisch.

(57) Verfahren zur Abtrennung von Schwefelsäure aus dem bei der Sulfoxidation von Paraffinen anfallenden Reaktionsgemisch, dadurch gekennzeichnet, daß man das Reaktionsgemisch mit einem organischen Amin-Sulfat versetzt und die sich dabei abscheidende Phase aus Wasser und Schwefelsäure abtrennt,

die verbleibende Alkansulfonsäure und Alkansulfonsäure- Amin-Salz enthaltende Phase mit Alkalihydroxid in einer solchen Menge versetzt, die ausreicht, um die Alkansulfonsäure und das Alkansulfonsäure-Amin-Salz unter Freisetzung des organischen Amins in das Alkali-Alkansulfonat Salz zu überführen,

durch Wasserdampfdestillation aus diesem Gemisch das Paraffin zusammen mit dem freien organischen Amin abtrennt,

das freie organische Amin von dem Paraffin durch Extraktion mit Schwefelsäure abtrennt und das dabei erhaltene organische Amin-Sulfat wieder zu Beginn des gesamten Verfahrens zur Abtrennung der Schwefelsäure einsetzt.

EP 0 089 653 A2

Croydon Printing Company Ltd.

Verfahren zum Abtrennen von Schwefelsäure aus dem bei der Sulfoxidation von Paraffinen anfallenden Reaktionsgemisch

Bei der Sulfoxidation von linearen Paraffinen nach dem Licht-Wasser-Verfahren, d.h. mit $SO_2$ und $O_2$ in Gegenwart von Wasser und mit UV-Licht, entsteht nach der Abtrennung von ungelöstem Paraffin eine Mischung aus Paraffin, Wasser, Alkansulfonsäure und Schwefelsäure. Die Zusammensetzung dieser Mischung ist abhängig von der Kettenlänge des eingesetzten Paraffins. Bei der Sulfoxidation eines Paraffins der Kettenlänge $C_{12}$-$C_{18}$ und einem Maximum bei $C_{14}$-$C_{17}$, erhält man beispielsweise nach dem Austreiben von $SO_2$ eine Mischung etwa folgender Zusammensetzung: 37,5 % Wasser, 32 % Paraffin, 23 % Alkansulfonsäure und 7,5 % Schwefelsäure.

Diese Mischung ist eine völlig klare Lösung und soll in den folgenden Ausführungen als Extrakt bezeichnet werden. Es handelt sich dabei wahrscheinlich um eine Mikroemulsion von Paraffin in der wäßrigen Schwefelsäure, wobei die Alkansulfonsäure als Solubilisierungsmittel wirkt.

Für die Gewinnung von reinem Alkansulfonsäure-Na-Salz, das als leicht biologisch abbaubares Tensid große Bedeutung besitzt, ist die Anwesenheit von Schwefelsäure sehr störend, da bei der Neutralisation ein entsprechender Anteil Natriumsulfat entsteht.

Es ist deshalb in der Vergangenheit immer wieder der Versuch unternommen worden, den Extrakt so zu beeinflussen, daß eine Abtrennung der Schwefelsäure erfolgt. So ist z.B. bekannt, daß eine Erwärmung des Extraktes auf ca. 90°C zu einer teilweisen Abtrennung der Schwefelsäure führt. Die technisch erreichbare Abtrennung liegt jedoch nur bei ca. 35 % Gesamtmenge $H_2SO_4$. Es ist ebenso

bekannt, dem Extrakt schwach polare Alkohole mit mindestens 5 Kohlenstoffatomen zuzusetzen, um eine Entmischung in eine organische Phase, welche die Gesamtheit der Sulfonsäure enthält und in eine wäßrige Phase, die fast die gesamte Schwefelsäure enthält, herbeizuführen. Der Nachteil bei diesem Verfahren liegt einerseits in der großen Menge an Alkohol, die dem Extrakt zugesetzt werden muß (zwischen 20-80 g Alkohol pro 100 g Extrakt) und andererseits in der schwierigen Trennung des Alkohols vom Paraffin.. Es ist schließlich bekannt, dem Extrakt schwach polare organische Lösungsmittel zuzusetzen wie Äther, Ketone, Ester und aliphatische Ketoester. Der Nachteil auch bei diesem Verfahren liegt in der großen Menge an Lösungsmitteln zur Abtrennung der Schwefelsäure. Diese Einsatzmenge wird in der Literatur mit 30-100 g pro 100 g Extrakt angegeben.

Es stellte sich daher die Aufgabe, eine möglichst quantitative Abtrennung der Schwefelsäure zu erreichen unter Zugabe möglichst geringer Mengen eines Hilfsmittels. Es wurde nun gefunden, daß man dieses Ziel erreichen kann, indem man dem bei der Sulfoxidation von Paraffinen anfallenden Reaktionsgemisch ein organisches Amin-Salz zugibt.

Gegenstand der Erfindung ist somit ein Verfahren zur Abtrennung von Schwefelsäure aus dem bei der Sulfoxidation von Paraffinen anfallenden Reaktionsgemisch, dadurch gekennzeichnet, daß man das Reaktionsgemisch mit einem organischen Amin-Sulfat versetzt und die sich dabei abscheidende Phase aus Wasser und Schwefelsäure abtrennt, die verbleibende Alkansulfosäure und Alkansulfonsäure-Amin-Salz enthaltende Phase mit Alkalihydroxid in einer solchen Menge versetzt, die ausreicht, um die Alkansulfosäure und das Alkansulfonsäure-Amin-Salz unter Freisetzung

des organischen Amins in das Alkali-Alkansulfonat Salz zu überführen, durch Wasserdampfdestillation aus diesem Gemisch das Paraffin zusammen mit dem freien organischen Amin abtrennt, das freie organische Amin von dem Paraffin durch Extraktion mit Schwefelsäure abtrennt und das dabei erhaltene organische Amin-Sulfat wieder zu Beginn des gesamten Verfahrens zur Abtrennung der Schwefelsäure einsetzt.

Bevorzugt nimmt man für die Abtrennung der Schwefelsäure die Sulfate von organischen Aminen der Formel

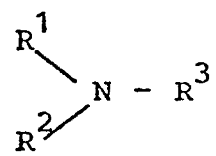

wobei $R^1$ $C_3$-$C_{18}$-Alkyl, $C_5$-$C_8$-Cycloalkyl, Phenyl, das durch 1 bis 3 $C_1$-$C_4$-Alkylgruppen oder eine $C_1$-$C_9$-Alkylgruppe substituiert sein kann, oder wenn $R^3$ eine Gruppe der Formel $-X-NR^1R^2$ darstellt, auch Wasserstoff, $R^2$ die gleiche Bedeutung hat wie $R^1$ und zusätzlich Wasserstoff, $R^3$ die gleiche Bedeutung hat wie $R^1$ und zusätzlich Wasserstoff oder eine Gruppe der Formel $-X-NR^1R^2$ und X Phenylen oder $C_8$-$C_{12}$-Alkylen bedeutet. Besonders bevorzugt sind solche Amine der obigen Formel, die nur Alkyl- bzw. Alkylengruppen, insbesondere geradkettige Alkyl- bzw. Alkylengruppen enthalten.

Geeignete Amine dieser Art sind aliphatische Mono-, Di- und Trialkylamine, wobei die Alkylreste sowohl geradkettig wie auch verzweigt sein können. Ebenso geeignet sind entsprechende cycloaliphatische Mono-, Di- und Trialkylamine. Auch aromatische Amine wie z.B. Xylidin oder Äthylanilin zeigen die gewünschte Wirkung ebenso wie auch längerkettige aliphatische Diamine.

Eine besonders gute Wirksamkeit zeigen die Amin-Sulfate folgender Amine:
Hexylamin, Heptylamin, Octylamin, Diisobutylamin, Dibutylmethylamin, Tripentylamin, Trihexylamin, Cyclohexylamin, Dicyclohexylamin, Tricyclohexylamin, Diaminooctan-1,8-Anilin, o-Toluidin, p-Toluidin, Xylidin, 2-Äthylanilin, o-Diaminobenzol, Aminodiphenyl und p-Nonylanilin.

Das erfindungsgemäße Verfahren zeichnet sich dadurch aus, daß am Ende des gesamten Prozesses eine wäßrige Lösung eines organischen Amin-Sulfats anfällt und daß dieses Amin-Sulfat zu Beginn des Prozesses erneut eingesetzt werden kann. Es versteht sich, daß man, um dieses Kreislaufverfahren erstmals in Gang zu setzen, anstelle des Amin-Sulfats ebenso auch das entsprechende freie Amin nehmen kann.

Diese Amine bzw. die entsprechenden Sulfate werden in einer Menge von 1 bis 5, vorzugsweise 2 bis 4 Gewichtsteilen, berechnet als freies Amin, zu 100 Gewichtsteilen des rohen Sulfonierungsgemisches (Extrakt) zugegeben. Zuvor wird der Extrakt noch von darin gelösten Restmengen an $SO_2$ befreit. Die Abscheidung der Unterphase aus Wasser und Schwefelsäure erfolgt unter Zugabe des Amins bzw. Amin-Sulfats schon bei Raumtemperatur. Um eine möglichst vollständige Abtrennung der Schwefelsäure zu erreichen, ist es vorteilhaft, den Extrakt auf ca. 20 bis 130, insbesondere 90 bis 95°C aufzuheizen. Nach der Zugabe des Amin-Sulfats bzw. Amins läßt man das Gemisch noch ca. 30 bis 60 Minuten stehen und trennt dann die entstandene Unterphase ab, die aus ca. 20 %iger wäßriger Schwefelsäure besteht und praktisch die gesamte, ursprünglich im Extrakt vorhandene Menge an Schwefelsäure enthält. Diese Schwefelsäure kann in einem gesonderten

Verfahren weiter aufgearbeitet werden. Die zurückbleibende obere Phase besteht aus dem nicht umgesetzten Rest-Paraffin und der Alkansulfonsäure, wobei ein Teil dieser Alkansulfonsäure in Form ihres Salzes mit dem als Phasentrennmittel zugegebenen Amin vorliegt. Da in der Praxis ein reines Alkansulfonat-Na Salz gefordert wird, stellte sich die Aufgabe, das eingesetzte Amin quantitativ aus dem Sulfonat zu entfernen und wirtschaftlich so aufzuarbeiten, daß es für eine weitere Trennung von Schwefelsäure aus dëm Extrakt eingesetzt werden kann.

Zu diesem Zweck wird zunächst zu der Oberphase, die man nach der Abtrennung der Schwefelsäure erhält und die Paraffin, freie Alkansulfonsäure und Alkansulfonsäure-Ammoniumsalz enthält, soviel Mol Natriumhydroxid zugegeben, wie der molaren Menge an freier Alkansulfonsäure und Alkansulfonsäure-Ammoniumsalz insgesamt entspricht.

Dies erreicht man, indem man soviel Natronlauge zugibt, bis der pH-Wert erreicht wird, der dem pH-Wert entspricht, den das freie Amin für sich allein in wäßriger Lösung zeigt (z.B. pH 10,5 bei Tributylamin). Dadurch wird das Amin von der Natronlauge aus der Salzbildung mit der Alkansulfonsäure verdrängt und freigesetzt. Anschließend wird dieses Gemisch einer Wasserdampfdestillation (Strippung) unterworfen bis zu einer Temperatur von ca. 250°C, wobei das noch vorhandene Paraffin zusammen mit dem freien organischen Amin abgetrennt wird. Die Temperatur von 250°C genügt in der Regel, um den Restparaffingehalt im Alkansulfonat unter 1 % zu senken. Das Amin-Sulfat bzw. Amin wird so gewählt, daß der Siedepunkt des freien Amins nicht höher als 300°C, vorzugsweise zwischen 150 und 250°C liegt. Durch diese beiden Maßnahmen, nämlich Einhaltung des pH-Wert in der angegebenen Weise und Auswahl eines Amins mit einem geeigneten

Siedepunkt ist gewährleistet, daß im Endprodukt nach der Aufarbeitung kein Amin oder Ammoniumsalz mehr nachweisbar ist.

Das gestrippte Paraffin muß aus Kostengründen wieder direkt der Sulfoxidation zugeführt werden. Es enthält das gesamte organische Amin, im Strippwasser selbst sind nur noch Spuren von Amin enthalten (weniger als 0,1 Gew.-%), da das Amin durch das Paraffin aus dem Wasser extrahiert wird.

Um die Sulfoxidation des Paraffins nicht zu erschweren, muß aber eine Trennung von Paraffin und Amin vorgenommen werden.
Dies geschieht, indem man dem Gemisch aus organischem Amin und Paraffin einen Teil der zu Beginn des gesamten Prozesses abgetrennten ca. 20 %igen Schwefelsäure zugibt.

Bei dieser Extraktion des Amins aus dem Paraffin geht man technisch so vor, daß man die wäßrige Schwefelsäure über einen Mischer dem Paraffin zudosiert und dann über einen nachgeschalteten Abscheider als Oberphase reines Paraffin und als Unterphase die Aminsalzlösung abzieht.
Die Menge an Schwefelsäure ist variabel. Als Obergrenze ergibt sich ein Verhältnis von 1 Mol Schwefelsäure (100 %ig) pro 2 Mol Amin im Paraffin. Man kann jedoch beliebig viel mehr Schwefelsäure zudosieren, als Amin vorhanden. Aus Gründen einer günstigen Raum-Zeitausbeute wird man aber ein Verhältnis von 1 Mol Schwefelsäure (100 %ig) auf 1,5 - 1,8 Mol Amin im Paraffin wählen oder 10 bis 40 Gew.-%, bezogen auf das Paraffin-Amin-Gemisch, der 20 %igen Schwefelsäure. Das so gereinigte Paraffin enthält noch einen Anteil an Amin von weniger als 0,1 % und kann in die Sulfoxidation zurückgeführt werden.

Die andere Phase, die aus der wäßrigen, schwefelsauren Lösung des organischen Amin-sulfats besteht, wird wieder dazu benutzt, aus dem Extrakt die Schwefelsäure abzutrennen. Diese saure Aminsalzlösung wird dem frischen Extrakt in einer solchen Menge zudosiert, daß das oben beschriebene optimale Verhältnis von Extrakt zu Amin (gerechnet als freies Amin) gegeben ist.

Es hat sich überraschenderweise gezeigt, daß diese Menge an Schwefelsäure, die durch die Zugabe des organischen Amins in Form des Sulfats wieder in den Extrakt eingebracht wird, im Verlauf des gesamten Abtrennprozesses ebenso wieder quantitativ abgetrennt wird. Die Amine bzw. Aminsalze sind somit in der Lage, aus dem Extrakt Schwefelsäure abzutrennen, unabhängig davon, ob sie als freie Base oder in Form ihrer Salze eingesetzt werden. In der Oberphase des Extrakts verbleibt nach Zugabe der Amine bzw. Aminsalze nur eine geringe Restmenge an Schwefelsäure (ca. 1-2 %), die allein abhängig ist von der eingesetzten Menge an Amin, nicht aber von der vorher eingebrachten Menge an Schwefelsäure durch das Aminsulfat. Dadurch ist es möglich, das Amin - wie oben beschrieben, ohne aufwendige und teure Trennoperationen im Kreis zu fahren, wobei die Verluste vernachlässigbar klein sind.

Das erfindungsgemäße Verfahren insgesamt zeichnet sich besonders dadurch aus, daß man eine vollständige Abtrennung der Schwefelsäure erreicht, wobei aber die hierzu benötigte Menge an Amin etwa um den Faktor 10 niedriger ist als bei den bisher zu diesem Zweck schwach polaren Lösungsmitteln. Das Verfahren wird insbesondere zur Trennung von rohen Sulfonierungsgemischen angewandt, die aus der Sulfoxidation von n-Paraffinen mit 7 bis 30, vorzugsweise 10 bis 20 C-Atomen stammen.

Beispiel 1

Zu 300 Gewichtsteilen von einem entgasten Extrakt mit folgender Zusammensetzung:

| | |
|---|---|
| Wasser: | 37,5 % |
| Paraffin: | 32 % |
| Alkansulfonsäure: | 23 % |
| Schwefelsäure: | 7,5 % |

gibt man unter Rühren 9 g Gew.-Teile Di-n-pentylamin bei 95°C und rührt 5 Minuten nach. Anschließend hält man die Mischung etwa 45 Minuten in einem beheizten Scheidetrichter, wobei sich der Extrakt in zwei Phasen trennt. Danach läßt man die farblose wäßrige Schwefelsäure als Unterphase ab. Man erhält 105 Gewichtsteile einer ca. 20 %igen Schwefelsäure, deren Gehalt titrimetrisch bestimmt wird. Bezogen auf die im Extrakt vorhandene Schwefelsäure werden 92 % abgetrennt.

Als Oberphase erhält man 204 Gewichtsteile einer Mischung aus

| | |
|---|---|
| Wasser: | 14,6 % |
| Paraffin: | 47 % |
| Alkansulfonsäure: | 33,6 % davon 0,057 Molprozent in Form des Di-n-pentylammoniumsalzes |

Diese Mischung wird mit ca. 25 g Gewichtsteilen 50 Gew.-%-iger Natronlauge auf pH 12,0 gestellt und anschließend einer Strippung mit überhitztem Wasserdampf (280 - 300°C) unterworfen, bis zu einer Innentemperatur von 250°C.

Das gestrippte Paraffin (ca. 105 Gewichtsteile) wird vom Strippwasser über einen Abscheider getrennt und anschließend mit ca. 18 Gewichtsteilen der vorher abgetrennten 20 %igen Schwefelsäure ausgeschüttelt (Molverhältnis $H_2SO_4$ : Amin = 1 : 1,58). Nach ca. 5 Minuten Abtrennzeit wird die wäßrige Unterphase abgelassen und der Gehalt von Di-n-pentylamin in der Schwefelsäure analytisch bestimmt. Es zeigt sich, daß die eingesetzten 9 Gewichtsteile Amin innerhalb der Meßgenauigkeit quantitativ wieder in der Schwefelsäurephase zu finden waren. Die so erhaltenen 24 Gewichtsteile schwefelsaures Di-n-pentylammoniumsalz in Wasser werden erneut 300 Gewichtsteilen eines Extraktes obiger Zusammensetzung zugegeben und man arbeitet wie oben angegeben weiter auf. Man erhält jetzt ca. 120 Gewichtsteile einer ca. 20 %igen Schwefelsäure, während der Restgehalt an Schwefelsäure in der Oberphase dem Gehalt bei Zugabe von reinem Di-n-pentylamin entspricht.

Beispiel 2

Zu 300Gewichtsteilen eines Extraktes gemäß Beispiel 1 gibt man 9 Gewichtsteile Tri-n-butylamin und arbeitet entsprechend dem Beispiel 1 weiter auf.

Nach Abtrennung der wäßrigen Schwefelsäure erhält man ca. 210 Gewichtsteile der Oberphase, welche mit 25,5 Gewichtsteilen 50 %iger Natronlauge auf pH 10,5 gestellt wird. Anschließend wird das Paraffin und das freigesetzte Amin abgestrippt und vom Strippwasser abgetrennt. Man erhält ca. 105 Gewichtsteile Paraffin + Amin, welche mit 14 Gewichtsteilen der vorher abgetrennten 20 %igen Schwefelsäure ausgeschüttelt werden (Molverhältnis $H_2SO_4$ zu Amin = 1 : 1,73). Nach einer kurzen Phasentrennzeit wird die Unterphase abgelassen und der Gehalt an Tri-n-butylamin in der Schwefelsäure bestimmt. Es zeigt

sich wieder, daß praktisch die gesamte Menge an Tributylamin in die wäßrige Schwefelsäure zurückgeführt worden ist. Man kann diese Lösung, wie im vorigen Beispiel beschrieben, direkt wieder zu einer weiteren Extraktaufarbeitung verwenden.

Beispiel 3

Zu 300 Gewichtsteilen eines Extraktes gemäß Beispiel 1 gibt man 16 Gewichtsteile Di-n-butylamin und trennt die Schwefelsäure, wie oben beschrieben, ab. Man erhält 214 Gewichtsteile einer Oberphase, welche die Gesamtmenge Alkansulfonsäure enthält. Diese Oberphase wird mit 25,8 Gewichtsteilen 50 %iger Natronlauge auf pH 12 gestellt und mit überhitztem Wasserdampf gestrippt.

Man erhält ca. 110 Gewichtsteile einer Mischung Paraffin/Dibutylamin, welche mit 35 Gewichtsteilen einer 20 %igen Schwefelsäure ausgeschüttelt werden (Molverhältnis $H_2SO_4$ : Amin = 1 : 1,77). Auch in diesem Fall zeigt die Analyse, daß das Dibutylamin quantitativ in die Schwefelsäurephase überführt worden ist.

Die vorliegenden Beispiele zeigen die diskontinuierliche Verfahrensweise. Selbstverständlich läßt sich das erfindungsgemäße Verfahren auch auf kontinuierliche Fahrweise anwenden, wobei die Vorzüge noch deutlicher sichtbar werden.

PATENTANSPRÜCHE:

1. Verfahren zur Abtrennung von Schwefelsäure aus dem bei der Sulfoxidation von Paraffinen anfallenden Reaktionsgemisch, dadurch gekennzeichnet, daß man das Reaktionsgemisch mit einem organischen Amin-Sulfat versetzt und die sich dabei abscheidende Phase aus Wasser und Schwefelsäure abtrennt,

die verbleibende Alkansulfonsäure und Alkansulfonsäure-Amin-Salz enthaltende Phase mit Alkalihydroxid in einer solchen Menge versetzt, die ausreicht, um die Alkansulfonsäure und das Alkansulfonsäure-Amin-Salz unter Freisetzung des organischen Amins in das Alkali-Alkansulfonat-Salz zu überführen,

durch Wasserdampfdestillation aus diesem Gemisch das Paraffin zusammen mit dem freien organischen Amin abtrennt,

das freie organische Amin von dem Paraffin durch Extraktion mit Schwefelsäure abtrennt und das dabei erhaltene organische Amin-Sulfat wieder zu Beginn des gesamten Verfahrens zur Abtrennung der Schwefelsäure einsetzt.

2. Verfahren nach Anspruch 1, dadurch gekennzeichnet, daß die Amin-Sulfate in einer Menge von 1 bis 5 Gewichtsteilen, gerechnet als freies Amin, auf je 100 Gewichtsteile Extrakt eingesetzt werden.

3. Verfahren nach Anspruch 1, dadurch gekennzeichnet, daß man ein Amin verwendet, das einen Siedepunkt von bis zu 300°C aufweist.